# EUROPEAN PATENT APPLICATION

(11) **EP 4 755 320 A2**
(43) Date of publication of application: **10.06.2026**
(21) Application number: 26170420.9
(22) Date of filing: 29.11.2023
(51) Int. Cl.: A61B 17/00

(54) **EMBOLIC DEVICES AND DELIVERING SYSTEMS**

(30) Priority: 02.12.2022 US 202263429944 P
(62) Divisional of application: 23837050.6
(71) Applicant: Stryker European Operations Limited, Carrigtwohill, Co. Cork T45 HX08 (IE); STRYKER CORPORATION, Portage, MI 49002-9711 (US)
(72) Inventor: WALSHE, Donal J., Grenagh, T23 A074 (IE); BRENNAN, Eamonn E., Ballincollig, T12 KD0V (IE); SHEEHAN, Tommy, Ballinhassig, T12 W61F (IE); COOPER, Alan A., Kinsale, P17TF86 (IE); WALKINGSHAW, Jason R., Cloyne, P25RX78 (IE)
(74) Representative: Hauck Patent- und Rechtsanwälte PartmbB

(57) **Abstract**

A medical device comprises a stem sized for insertion into a lumen of a coil; a stopper coupled to, and/or extending from, the stem. The stopper is proximal to a proximal end of the coil when the stem is inserted into the lumen of the coil and a structure outside the lumen of the coil. The structure defining an opening is sized to accommodate a distal portion of a delivery wire, and the stem comprises a channel configured to accommodate a first part of a stretch-resistant member when a second part of the stretch-resistant member is disposed around the structure that defines the opening.

## Description

### FIELD

The field of the application relates to medical devices, and more specifically, to embolic devices and delivering systems for delivering embolic devices.

### BACKGROUND

An aneurysm is a dilation of a blood vessel that poses a risk to health from the potential for rupture, clotting, or dissecting. Rupture of an aneurysm in the brain causes stroke, and rupture of an aneurysm in the abdomen causes shock. Cerebral aneurysms are usually detected in patients as the result of a seizure or hemorrhage and can result in significant morbidity or mortality.

There are a variety of materials and devices which have been used for treatment of aneurysms, including platinum and stainless steel microcoils, polyvinyl alcohol sponges (Ivalone), and other mechanical devices. For example, embolic (or vaso-occlusion) devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel.

Embolic devices or implants are used for a wide variety of reasons, including treatment of intra-vascular aneurysms. Commonly used embolic devices include soft, helically wound coils formed by winding a platinum (or platinum alloy) wire strand about a "primary" mandrel. The coil is then wrapped around a larger, "secondary" mandrel, and heat treated to impart a secondary shape. For example, U.S. Pat. No. 4,994,069, issued to Ritchart et al., which is fully incorporated herein by reference as though set forth in full, describes a vaso-occlusive device that assumes a linear, helical primary shape when stretched for placement through the lumen of a delivery catheter, and a folded, convoluted secondary shape when released from the delivery catheter and deposited in the vasculature.

In order to deliver the embolic devices to a desired site in the vasculature, e.g., within an aneurysmal sac, it is well-known to first position a small profile, delivery catheter or "micro-catheter" at the site using a steerable guidewire. Typically, the distal end of the micro-catheter is provided, either by the attending physician or by the manufacturer, with a selected pre-shaped bend, e.g., 45°, 26°, "J", "S", or other bending shape, depending on the particular anatomy of the patient, so that it will stay in a desired position for releasing one or more embolic device(s) into the aneurysm once the guidewire is withdrawn. A delivery or "pusher" assembly is then passed through the micro-catheter, until an embolic device secured to a distal end of the pusher assembly is extended out of the distal end opening of the micro-catheter and into the aneurysm. The proximal end of the embolic device is typically secured to the distal end of the pusher assembly with an adhesive at what is known as a "major junction" of the embolic device delivery assembly.

Another embolic device delivery assembly major junction design is disclosed in U.S. Pat. No. 8,202,292, issued to Kellett, which is fully incorporated herein by reference as though set forth in full. The major junction includes a flat adapter connecting a delivery wire to an embolic (vaso-occlusive) coil. The delivery wire has a hook or "J" shape distal end configured to be received in an aperture in the proximal end of the adapter to attach the delivery wire to adapter. The embolic coil has windings that define openings configured to receive fingers in the distal end of the adapter to attach the embolic coil to the adapter. Consequently, the adapter facilitates attachment of the delivery wire to the embolic coil.

After the embolic device is delivered in the aneurysm, the embolic device is then released or "detached" from the end of the pusher assembly, typically by detaching a distal end of the pusher assembly. Then the pusher assembly is withdrawn back through the catheter. Depending on the particular needs of the patient, one or more additional occlusive devices may be pushed through the catheter and released at the same site.

One well-known way to release an embolic device from the end of the pusher assembly is through the use of an electrolytically severable junction, which is a small exposed section or detachment zone located along a distal end portion of the pusher assembly. The detachment zone is typically made of stainless steel and is located just proximal of the embolic device. An electrolytically severable junction is susceptible to electrolysis and disintegrates when the pusher assembly is electrically charged in the presence of an ionic solution, such as blood or other bodily fluids. Thus, once the detachment zone exits out of the catheter distal end and is exposed in the vessel blood pool of the patient, a current applied through an electrical contact to the conductive pusher completes an electrolytic detachment circuit with a return electrode, and the detachment zone disintegrates due to electrolysis. Other detachment mechanisms for releasing an embolic device from a pusher assembly include mechanical, thermal, and hydraulic mechanisms.

While major junctions secured with an adhesive and those including a flat adapter have performed well, connections between the delivery wire, the embolic coil, and the adapter can be improved. Accordingly, there remains a need for other systems and methods for securing an embolic device to a pusher assembly at a major junction.

US 2013211495 A1 describes a delivery system that comprises an implant comprising a socket at a proximal end of the implant; an elongate sleeve having a proximal section, a distal section slidably disposed within the socket, and a window between the proximal section and the distal section, the window extending through a wall of the sleeve. The device further comprises a core member having a proximal portion slidably received within the proximal section of the sleeve and a distal portion extending through the window to a space outside the sleeve and within the socket. The distal portion of the core member provides an interference fit with the distal section of the sleeve within the socket until the core member is withdrawn.

US 2009297582 A1 describes a device for in situ treatment of vascular or cerebral aneurysms comprises an occlusion device having a flexible, longitudinally extending elastomeric matrix member that assumes a non-linear shape to conformally fill a targeted site. The occlusion device comprises a flexible, longitudinally extending elastomeric matrix member, wherein the device assumes a non-linear shape capable of fully, substantially, or partially conformally filling a targeted vascular site.

US 2018206849 A1 describes a device that includes a self-expanding resilient permeable shell having a radially constrained state and an expanded state with a globular, axially shortened configuration. The permeable shell may be a single layer of braided elongate filaments having first and second ends that are secured at the proximal end of the permeable shell. The devices may also include permeable shells made of woven braided mesh having a variable mesh density, i.e., the average size of pores in one region are a different than the average size of pores in another region.

### SUMMARY

The invention is based on the task of providing an improved device of the type described above. The invention is set out in claims.

In accordance with the invention is a medical device provided in claim 1. Advantageous embodiments are specified in the dependent claims.

As an example, that may, if applicable, provide details to further specify embodiments claimed or described in this application a medical device is described that includes: a socket having one or more side wall(s) defining a cavity sized for receiving a plurality of braid wires, wherein the socket also has an end wall coupled to, and/or extending from, the one or more side wall(s); and a first anchor on an interior surface of the one or more side wall(s) of the socket, wherein the first anchor is configured to press against at least one of the braid wires after the socket has received the braid wires.

Optionally, the medical device further includes a loop coupled to the end wall of the socket, the loop defining a loop opening sized to accommodate a distal portion of a delivery wire.

Optionally, the medical device further includes the delivery wire, wherein the distal portion of the delivery wire is connected to a remaining portion of the delivery wire via a detachable link.

Optionally, the loop and the cavity are on opposite respective sides of the end wall.

Optionally, the one or more side wall(s) comprises a plurality of side walls.

Optionally, the one or more side wall(s) comprises only a single circumferential side wall defining the cavity.

Optionally, the medical device further includes the braid wires, wherein the braid wires are parts of a braided structure.

Optionally, the medical device is an embolic device, and wherein the braided structure is sized for insertion into an aneurysm.

Optionally, the braid wires comprise at least 3 braid wires.

Optionally, the medical device further includes an adhesive connecting to the socket and the braid wires.

Optionally, the first anchor comprises a strut or a barb.

Optionally, the medical device further includes a second anchor on the interior surface of the one or more side wall(s) of the socket.

Optionally, the first anchor and the second anchor are in a row oriented in a direction that is parallel to a longitudinal axis of the socket.

Optionally, the socket is made from HTL, a BIO polymer, palladium, a bio-compatible material, or a 3D-printing material.

Optionally, the medical device further includes one or more additional anchors, wherein the first anchor and the one or more additional anchors are configured to allow the braid wires to be inserted into the socket with an insertion force, and to resist a removal force to prevent the braid wires from being removed from the socket, wherein the removal force is higher than the insertion force.

As another example, that may, if applicable, provide details to further specify embodiments claimed or described in this application a medical device is described that includes: a stem sized for insertion into a lumen of a coil; a stopper coupled to, and/or extending from, the stem, wherein the stopper is proximal to a proximal end of the coil when the stem is inserted into the lumen of the coil; and a structure outside the lumen of the coil, the structure defining an opening sized to accommodate a distal portion of a delivery wire, and wherein the stem comprises a channel configured to accommodate a first part of a stretch-resistant member when a second part of the stretch-resistant member is disposed around the structure that defines the opening.

Optionally, the medical device further includes the stretch-resistant member.

Optionally, the medical device further includes the delivery wire, wherein the distal portion of the delivery wire is connected to a remaining portion of the delivery wire via a detachable link.

Optionally, the medical device further includes a braided structure coupled to the coil, wherein the braided structure is sized for insertion into an aneurysm.

Optionally, the medical device further includes the coil.

As another example, that may, if applicable, provide details to further specify embodiments claimed or described in this application a medical device is described that includes: a stem sized for insertion into a lumen of a coil; a stopper coupled to, and/or extending from, the stem, wherein the stopper is proximal to a proximal end of the coil when the stem is inserted into the lumen of the coil; and a loop proximal to the stopper, the loop defining a loop opening sized to accommodate a distal portion of a delivery wire, and to accommodate a part of a stretch-resistant member.

Optionally, the stem comprises a recess to accommodate another part of the stretch-resistant member.

Optionally, the medical device further includes the stretch-resistant member.

Optionally, the medical device further includes the delivery wire, wherein the distal portion of the delivery wire is connected to a remaining portion of the delivery wire via a detachable link.

Optionally, the medical device further includes a braided structure coupled to the coil, wherein the braided structure is sized for insertion into an aneurysm.

Optionally, the medical device further includes the coil.

Other and further aspects and features will be evident from reading the following detailed description.

### DESCRIPTION OF THE DRAWINGS

The drawings illustrate the design and utility of embodiments, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only exemplary embodiments and are not therefore to be considered limiting in the scope of the claims.
**FIG. 1** illustrates a medical system having a catheter for delivering an embolic device.
**FIG. 2A** illustrates the medical system of **FIG. 1****,** particularly showing a distal segment of the embolic device being delivered out of the catheter.
**FIG. 2B** illustrates the medical system of **FIG. 1****,** particularly showing a proximal segment of the embolic device being delivered out of the catheter.
**FIGS. 3A-3B** illustrate a method of using the medical system of **FIG. 1****.**
**FIG. 4** illustrates an example of the embolic device of **FIG. 1****.**
**FIG. 5** illustrates an example of a component of the embolic device of **FIG. 1****.**
**FIGS. 6A-6C** illustrate the component of the embolic device of **FIG. 5****,** particularly showing the component coupled to braid wires.
**FIG. 7** illustrates a variation of the component of **FIG. 5****.**
**FIG. 8** illustrates another variation of the component of **FIG. 5****.**
**FIGS. 9****,** **10A,** and **10B** illustrate another example of a component of the embolic device of **FIG. 1****.**
**FIGS. 11A-11E** illustrate variations of the component of **FIG. 9****.**
**FIG. 12** illustrates another example of a component of the embolic device of **FIG. 1****.**
**FIGS. 13A-13C** illustrate the component of the embolic device of **FIG. 12****.**
**FIG. 14A** illustrates an example of a part of the embolic device of **FIG. 1****.**
**FIG. 14B** illustrates another example of a part of the embolic device of **FIG. 1****.**

### DETAILED DESCRIPTION

Various embodiments are described hereinafter with reference to the figures. It should be noted that the figures are not drawn to scale and that elements of similar structures or functions are represented by the same reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

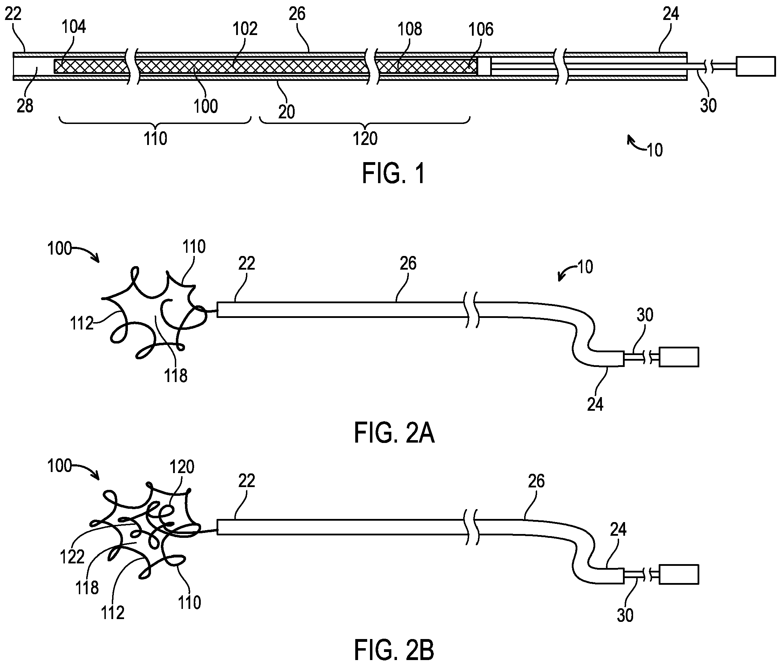

**FIG. 1** illustrates a medical device 10 having a catheter 20 for delivering an embolic device 100 in a body lumen. The catheter 20 has a distal end 22, a proximal end 24, and a catheter body 26 extending between the distal end 22 and the proximal end 24. The embolic device 100 is contained within a lumen 28 of the catheter 20. The catheter 20 further includes a delivery wire (e.g., a shaft) 30 located in the lumen 28 for pushing the embolic device 100 out of the lumen 28 of the catheter 20.

As shown in **FIG. 1****,** the embolic device 100 is made from an elongated member 102 having a distal end 104, a proximal end 106, and a body 108 extending between the distal end 104 and the proximal end 106. The embolic device 100 has a first segment 110 having a first linear configuration when located inside the catheter 20. The first segment 110 is configured to form a first three-dimensional structure 112 when the first segment 110 is delivered outside the catheter 20 **(****FIG. 2A****).**

As also shown in **FIG. 1****,** the embolic device 100 has a second segment 120 extending from the first segment 110. The second segment 120 is proximal with respect to the first segment 110. The second segment 120 has a second linear configuration when located inside the catheter 20. The second segment 120 is configured to form a second three-dimensional structure 122 when the second segment 120 is delivered outside the catheter 20 **(****FIG. 2B****).** In some embodiments, the first segment 110 and the segment 120 may be parts of a single structure that is formed to have the first segment 110 and the second segment 120. In other embodiments, the first segment 110 and the second segment 120 may be separate components that are connected together, e.g., via adhesive, weld, fusion, mechanical connector, etc. In either case, the first segment 110 and the second segment 120 may be considered as having or forming a unity configuration. In other cases, the embolic device 100 may have only one segment or more than two segments.

As shown in **FIG. 2A****,** the first three-dimensional structure 112 defines a cavity 118 after the first segment 110 forming the first three-dimensional structure 112 is delivered out of the catheter 20. Accordingly, after the first segment 110 has been delivered out of the catheter 20 into a body lumen, the first three-dimensional structure 112 provides the cavity 118 for accommodating a remaining part (e.g., at least a majority of the second segment 120 forming the second three-dimensional structure 122) of the embolic device 100.

In other cases, the segments 110, 120 of the embolic device 100 may not form respective three-dimensional structures 112, 122 that are in a nested-configuration. Instead, the embolic device 100 may have one or more segments forming a plurality of loops, wherein the loops form a three-dimensional structure to occupy a cavity of an aneurysm. In further cases, the embolic device 100 may be any implant that is capable of forming a three-dimensional structure for occupying a body cavity.

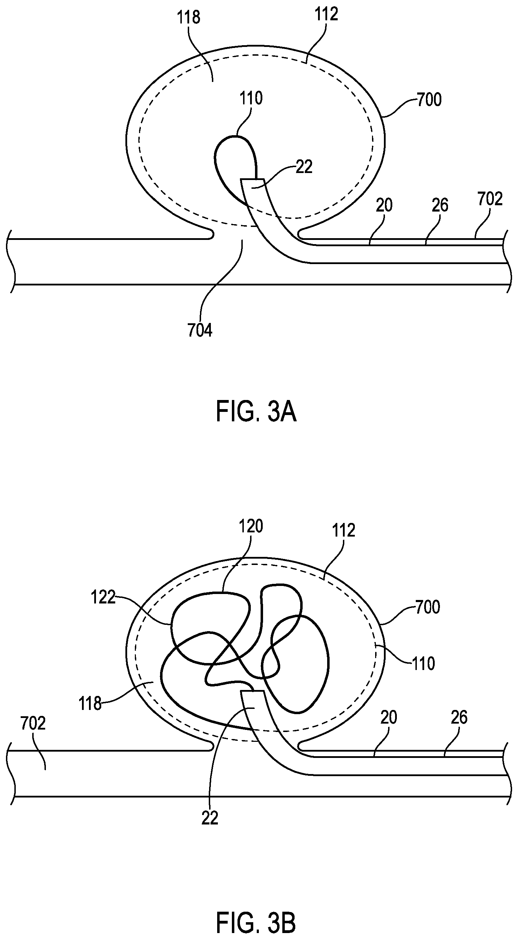

**FIGS. 3A-3B** illustrate a method of using the medical device 10 of **FIG.** 1 to treat an aneurysm 700. When using the medical device 10, the catheter 20 is first inserted into a blood vessel 702 of a patient through an incision. The catheter 20 is then advanced distally until the distal end 22 of the catheter 20 is at the aneurysm.

In some embodiments, the catheter 20 may be steerable. For example, the catheter 20 may include one or more steering wires configured to steer the distal end 22 of the catheter 20 in one or more directions. In other embodiments, the catheter 20 may not be steerable. Instead, a guidewire may first be used to access the target site. Then the catheter 20 may be disposed over the guidewire, and advanced distally using the guidewire. In such cases, the catheter 20 may include a separate channel for accommodating the guidewire.

After the distal end 22 of the catheter 20 is desirably placed, the shaft 30 (shown in **FIG. 1****)** is then advanced to push the embolic device 100 distally until the first segment 110 of the embolic device 100 is outside the catheter 20 **(****FIG. 3A****).** As shown in the figure, the first segment 110 forms the first three-dimensional structure 112 when the first segment 110 is unconfined outside the catheter 20. The first three-dimensional structure 112 has a shape that corresponds with an inner wall of the aneurysm such that the first three-dimensional structure 112 is positioned closely next to (e.g., against or within 0.5 mm from) the wall of the aneurysm. The first three-dimensional structure 112, represented schematically by the dashed line in **FIG. 3A****,** provides a frame defining the cavity 118 for accommodating the second segment 120 of the embolic device 100. As shown in the figure, the first three-dimensional structure 112 also provides a scaffolding across a neck 704 of an aneurysm 700, which assists in containing the second-dimensional structure 122 to be delivered into the cavity 118.

Next, the shaft 30 may be advanced further to push the second segment 120 of the embolic device 100 until the second segment 120 is out of the catheter 20 **(****FIG. 3B****).** As shown in the figure, the second segment 120 forms the second three-dimensional structure 122 when the second segment 120 is unconfined outside the catheter 20. The second three-dimensional structure 122 has a shape that allows it to fill at least some of the space in the cavity 118 defined by the first three-dimensional structure 112. As shown in the figure, the scaffolding across the neck 704 of the aneurysm provided by the first three-dimensional structure 112 prevents the second-dimensional structure 122 from escaping or falling out of the cavity 118 of the first three-dimensional structure 112 and out of the aneurysm.

In other cases, the embolic device 100 may not be configured to form multiple three-dimensional structures that are in the nested configuration. In such cases, as the shaft 30 is advanced to push the embolic device 100 out of the catheter 20, the embolic device 100 may form one or more three-dimensional structures that fill a cavity of the aneurysm in a non-nested configuration. For example, the one or more three-dimensional structures formed by the embolic device 100 may fill the aneurysm cavity from a distal end of the aneurysm towards a proximal end of the aneurysm, or may fill the aneurysm cavity in a random configuration.

In some embodiments, the distal end of the delivery wire (e.g., shaft) 30 may be mechanically connected to the proximal end of the embolic device 100 via a disintegratable link, such as a link that can be disintegrated in response to application of a current.

In some embodiments, multiple embolic devices 100 may be provided with different respective lengths. In such cases, before one of the embolic devices 100 is selected for treating an aneurysm, a doctor may measure a size of the aneurysm to be treated. For example, the doctor may perform measurement using one or more images of the aneurysm to determine the size of the aneurysm. The size may be a cross-sectional dimension of the aneurysm, a cross-sectional area of the aneurysm, a volume of the aneurysm, etc. After a size of the aneurysm has been determined, one of the embolic devices 100 may then be selected based on the size of the aneurysm. For example, a longer embolic device 100 may be selected to occlude a larger aneurysm.

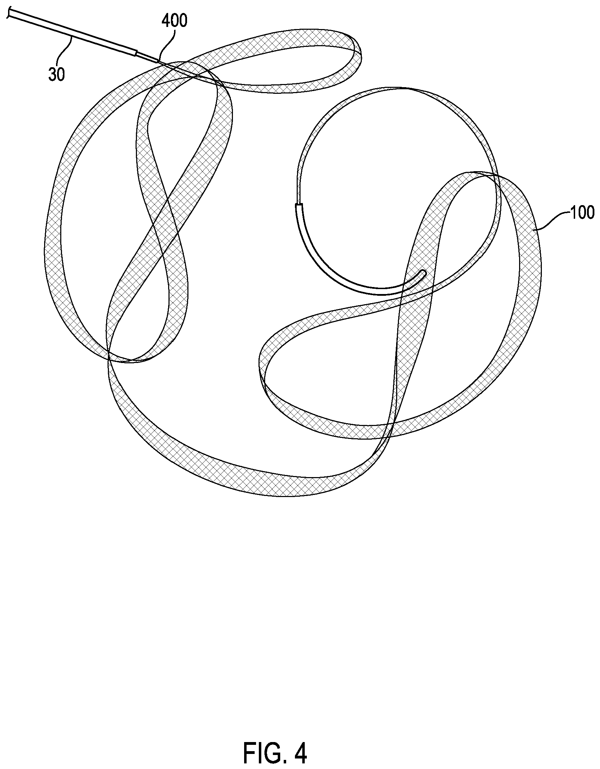

**FIG. 4** illustrates an example of the embolic device 100 of **FIG. 1****.** The embolic device 100 is a braided structure made from a plurality of braid wires. The embolic device 100 is detachably coupled to a portion of a delivery wire 30 via a joint 400. In some cases, the embolic device 100 may optionally include a coil coupled between the braid wires of the braided structure and the delivery wire 30. In other cases, the embolic device 100 may not include such coil. In further cases, the embolic device 100 may not include a braided structure. Instead, the embolic device 100 may have a coil that is configured to form a three-dimensional structure for occupying an aneurysm. Also, in some cases, the joint 400 may be considered to be a part of the delivery wire 30, and/or may be formed from a same material as the delivery wire 30.

The delivery wire 30 may be coupled to the embolic deice 100 using different techniques in different embodiments. For example, in some cases, the embolic device 100 may include a component that connects the braid wires of the embolic device 100 with the delivery wire 30.

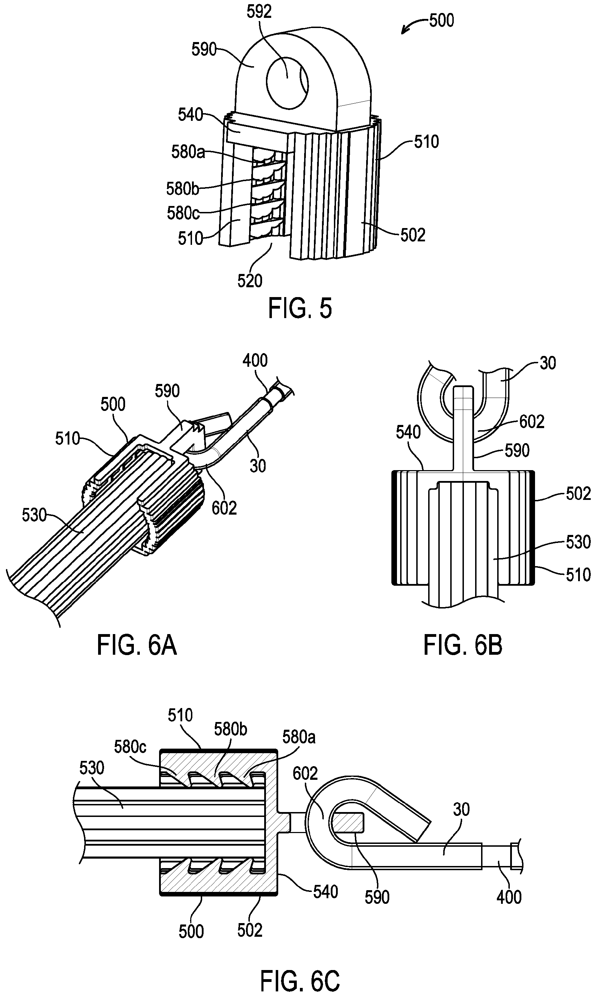

**FIG. 5** and **FIGS. 6A****-6C** illustrate an example of a component 500 of the embolic device 100 of **FIG. 1****.** The component 500, which may be considered an example of a medical device, is configured to interface between braid wires of the embolic device 100 and the delivery wire 30. The component 500 includes a socket 502 having one or more side wall(s) 510 defining a cavity 520 sized for receiving a plurality of braid wires 530. The socket 502 also has an end wall 540 coupled to, and/or extending from, the one or more side wall(s) 510. The socket 502 further has a first anchor 580a on an interior surface of the one or more side wall(s) 510 of the socket 502. The first anchor 580a is configured to press against at least one of the braid wires 530 after the socket 502 has received the braid wires 530.

In the illustrated example, the socket 502 has two side walls 510 defining the cavity 520 for receiving the braid wires 530. In other cases, the socket 502 may have more than two side walls 510 that define the cavity 520. In further example, the one or more side wall(s) 510 may comprise only a single circumferential side wall 510 defining the cavity 520.

In some cases, the braid wires 530 may be inserted into the cavity 520 of the socket 502 with an insertion force Fi, and the socket 502 is configured to grip the braid wires 530 so that the socket 502 can resist a removal force Fr to prevent the braid wires 530 from being removed from the socket. The removal force Fr may be higher than the insertion Force Fi in some cases.

Optionally, the component 500 may further include one or more additional anchors 580 (e.g., anchor 580b and/or anchor 580c), wherein the first anchor 580a and the one or more additional anchors are configured to allow the braid wires 530 to be inserted into the socket 502 with an insertion force, and to resist a removal force to prevent the braid wires 530 from being removed from the socket 502. The removal force may be higher than the insertion force in some cases.

As shown in **FIG. 5** and **FIG. 6C****,** the anchors 580a-580c are in a row oriented in a direction that is parallel to a longitudinal axis of the socket 502. In other cases, the anchors 580a-580c may be aligned in a row that is not parallel to the longitudinal axis of the socket 502. Also, in further cases, the anchors 580a-580c may not be aligned in a row (e.g., a rectilinear row). Instead, the anchors 580a-580c may have a staggered configuration. Also, in further cases, the socket 502 may have more than three anchors 502, or fewer than three anchors 502. For example, in other cases, the socket 502 may have two anchors 502 or only a single anchor 502 (e.g., the first anchor 502a).

In some cases, the first anchor 580a, the second anchor 580b, or the third anchor 580c may comprise a strut or a barb. However, the anchors 580a-580c should not be limited to these examples. In other cases, each of the anchors 580a-580c may be any structural component that allows the braid wires 530 to be inserted into the cavity 520 of the socket 502, while contributing to resisting against a removal force being applied to pull the braid wires 530.

The braid wires 530 are parts of a braided structure of the embolic device 100. The braided structure of the embolic device is sized for insertion into an aneurysm. In some cases, the braid wires 530 may comprise at least 3 braid wires. In other cases, the braid wires 530 may comprise more than 3 braid wires, or fewer than 3 braid wires. For examples, in other cases, the braid wires 530 may comprise 4, 5, 6, 7, 8, 9, 10, 11, 12, 14, 16, 18, 20, 22, 24, 26, 28, 30 braid wires.

As shown in **FIGS. 5** and **6A****-6C**, the component 500 further includes a loop 590 coupled to the end wall 540 of the socket 502. The loop 590 defines a loop opening 592 sized to accommodate a distal portion 602 of the delivery wire 30. The distal portion 602 of the delivery wire 30 has a curvilinear configuration, and is anchored against the loop 590 while the distal portion 602 is accommodated in the loop opening 592. As shown in the figures, the loop 590 and the cavity 520 are on opposite respective sides of the end wall 540.

In some cases, the socket 502 and the loop 590 may be formed together during a manufacturing process. For example, the socket 502 and the loop 590 may be formed together via a molding process, or a printing process. Alternatively, the socket 502 and the loop 590 may be formed separately, and then connected to each other via an adhesive.

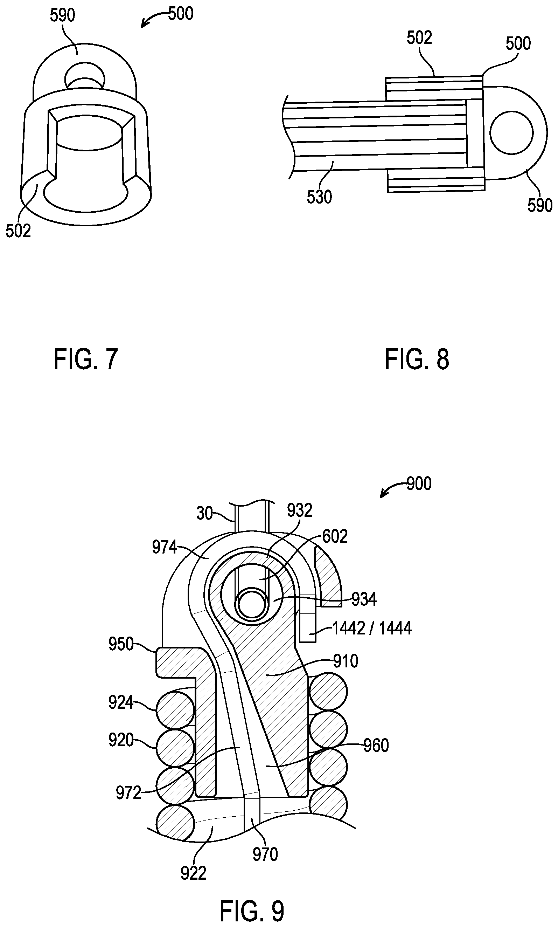

The socket 502 and the loop 590 may be made from a same material, or different respective materials. For examples, the socket 502 and/or the loop 590 may be made from polymer(s), metal(s), or alloy(s) (such as HTL, a BIO polymer, palladium, etc.). In some cases, the socket 502 and/or the loop 590 may be made from a bio-compatible material, or a 3D-printing material. **FIG. 7** illustrates a variation of the component 500 of **FIG. 5****,** particularly showing the component 500 being made from a polymer. **FIG. 8** illustrates another variation of the component 500 of **FIG. 5****,** particularly showing the component 500 being made from a metal.

In some cases, the delivery wire 30, or at least the distal portion 602 of the delivery wire 30, may be considered to be a part of the component 500. The distal portion 602 of the delivery wire 30 is connected to a remaining portion of the delivery wire 30 via a detachable link 400. In some cases, the detachable link 400 may also be considered to be a part of the component 500. In other cases, the distal portion 602 and/or the detachable link 400 is not a part of the component 500.

Also, in some cases, the component 500 may optionally include the braid wires 530. In such cases, the braid wires 530 are parts of the component 500. In other cases, the braid wires 530 are not parts of the component 500. Because the braid wires 530 are parts of a braided structure forming the embolic device 100, in some cases, the component 500 may optionally include the braided structure or the embolic device 100.

In some cases, the side wall(s) 510 and the end wall 540 of the socket 502 may be integrally formed together so that they have an unity or monolithic configuration. Also, in some cases, the loop 590 may also be integrally formed together with the end wall 540 and the side wall(s) 510 so that they have an unity or monolithic configuration. In other cases, the side wall(s) 510, the end wall 540, the loop 590, or any combination of the foregoing, may be individually formed, and may be secured to each other via an adhesive.

In addition, in some cases, the component 500 may optionally further include an adhesive connecting to the socket 502 and the braid wires 530. The adhesive may be disposed between the wall(s) 510 of thee socket 502 and the braid wires 530. Alternatively, the adhesive may be a blob that encompasses the socket 502 and ends of the braid wires 530.

As mentioned, the component 500 may be considered a medical device. The medica device may include only the socket 502 and the loop 590. Alternatively, the medical device may optionally include the braid wires 530, the braid structure formed by the braid wires 530, the embolic device that comprises the braid structure, the distal portion 602 of the delivery wire 30, the delivery wire 30, the detachable link 400, or any combination of the foregoing. Thus, as used in this specification, the term "medical device" is not limited to a finished or a completed commercial product, and may refer to one or more components of a product.

It should be noted that the component / medical device 500 is advantageous because it provides a structural interface that connects the braid wires 530 with the distal portion 602 of the delivery wire 30. The component / medical device 500 obviates any need to use a coil as an interface between the braid wires 530 and the delivery wire 30. Also, the component / medical device 500 allows the braid wires 530 to be easily and efficiently connected to the delivery wire 30 via a few simple steps. In one example, the braid wires 530 may be inserted into the cavity 520 of the socket 502. The anchor(s) 580 at the interior wall(s) of the socket 502 helps secure the braid wires 530 relative to the socket 502. Optionally, adhesive may be applied to enhance the securement between the braid wires 530 and the socket 502. Next, the distal portion 602 of the delivery wire 30 may be inserted into the loop opening 592 of the loop 590, and may anchor against the loop 590. In some cases, the adhesive may be applied after the distal portion 602 of the delivery wire 30 is anchored to the loop 590. The adhesive may be a blob that encompasses or surrounds that socket 502, the loop 590, a part of the braid wires 530, a part of the distal portion 602 of the delivery wire 30, any combination of the foregoing, or all of the foregoing.

It should be noted that the component 500 is not limited to having the configuration shown in the above example, and that the component 500 may have other configurations in other cases. For example, in other cases, the loop 590 may have a thicker configuration, and may have the same width as the end wall 540. Also, in other cases, the end wall 540 may not have a planar configuration like that shown in the above example, and may instead have a block-like configuration. Thus, as used in this specification, the term "wall" is not limited to a structure having a thin planar configuration, and may cover structures having other shapes, and/or may cover any structure that is between two spaces.

In the above example of **FIG. 5****,** the component 500 is specifically configured to couple to the embolic device 100 that has the braided wires 530 and without any coil coupled to the proximal ends of the braided wires 530. In other cases, the embolic device 100 may have a coil coupled to the proximal ends of the braided wires 530. In such cases, a component may be provided that interfaces between the coil and the delivery wire 30.

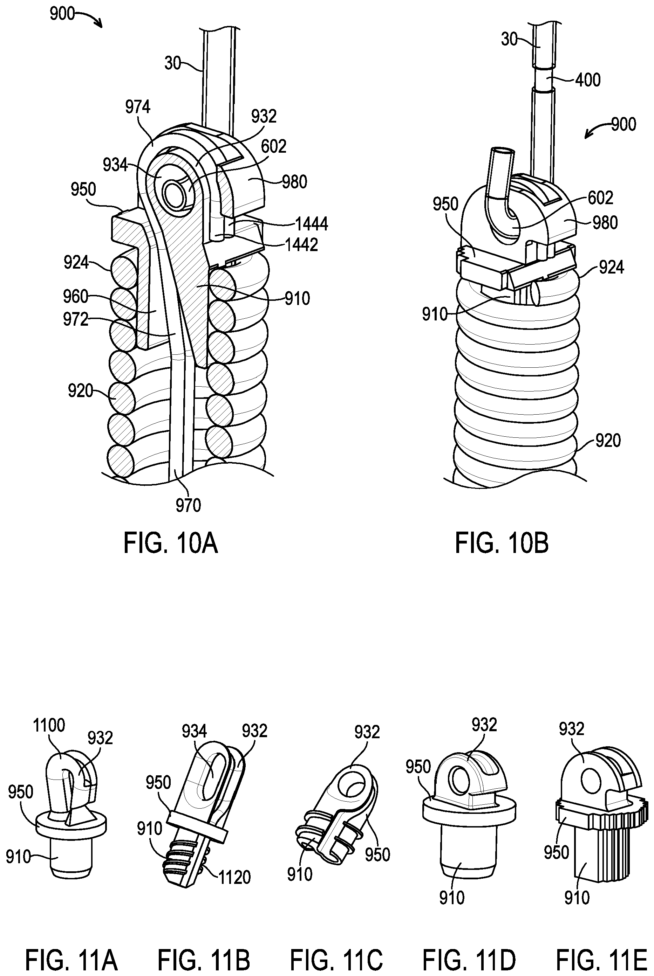

**FIGS. 9****,** **10A,** and **10B** illustrate another example of a component 900 of the embolic device 100 of **FIG. 1****.** The component 900, which may be considered an example of a medical device, is configured to interface between a coil of the embolic device 100 (in the cases in which the embolic device 100 includes the coil) and the delivery wire 30. The component 900 includes a stem 910 sized for insertion into a lumen 922 of a coil 920. The component 900 also includes a stopper 950 coupled to, and/or extending from, the stem 910, wherein the stopper 950 is proximal to a proximal end 924 of the coil 920 when the stem 910 is inserted into the lumen 922 of the coil 920. The stopper 950 is configured to prevent the stem 910 from being inserted too deeply into the lumen 922 of the coil 920. The stopper 950 may have a surface configured to abut against an end loop of the coil 920 at the proximal end 924 of the coil 920. Alternatively, the stopper 950 may not abut against the end loop of the coil 920. Instead, the stopper 950 may be spaced away from the end loop, or may be coupled indirectly to the end loop, e.g., via another structural component, such as a disk, an adhesive, etc. The component 900 further includes a structure 932 located outside the lumen 922 of the coil 920. The structure 932 is proximal to the stem 910 and the stopper 950. The structure 932 defines an opening 934 sized to accommodate a distal portion 602 of the delivery wire 30. The stem 910 comprises a channel 960 configured to accommodate a first part 972 of a stretch-resistant member 970 when a second part 974 of the stretch-resistant member 970 is disposed around the structure 932 that defines the opening 934.

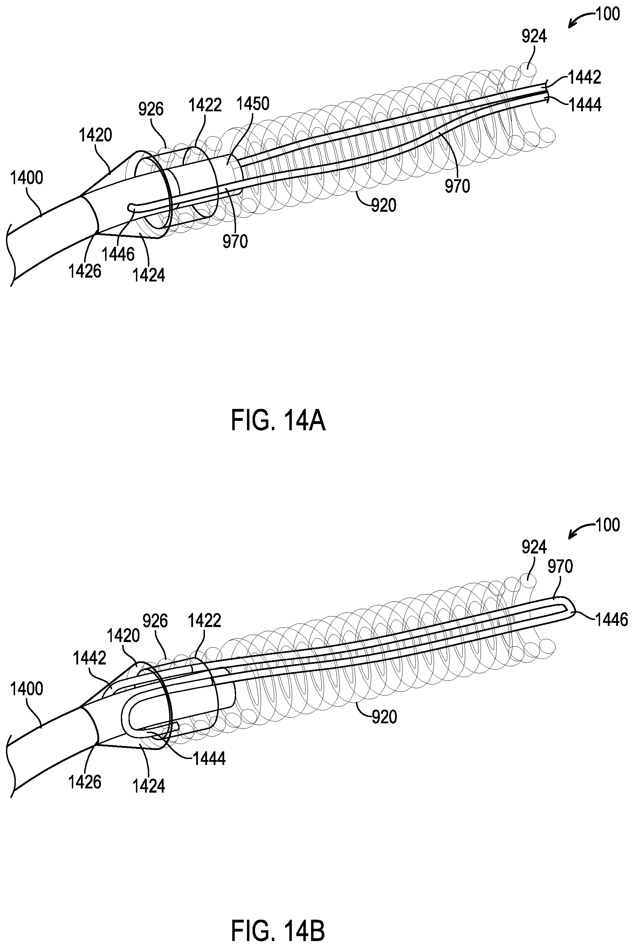

In the illustrated example, the component 900 has one channel 960 to accommodate the first part 972 of the stretch-resistant member 970, and the stretch-resistant member 970 forms a hook that terminates with an end 1442/1444 that is closer to the proximal end 924 of the coil 920 than to a distal end 926 of the coil 920 (like that shown in **FIGS. 10A** and **14A****).** The stretch-resistant member 970 may extend to the distal end 926 of the coil 920, form a loop there, and then extend back to the proximal end 924 of the coil 920. In such cases, both opposite ends 1442, 1444 of the stretch-resistant member 970 are located closer to the proximal end 924 of the coil 920 than to the distal end 926 of the coil 920 (like that shown in **FIGS. 10A** and **14A****).**

In other cases, the stretch-resistant member 970 form a loop 1446 around the structure 932, and may extend back towards the distal end 926 of the coil 920 (like that shown in **FIG. 14B**). In such cases, the component 900 may optionally include a second channel at the stem 910 for accommodating another segment (the returning part) of the stretch-resistant member 970. Alternatively, the same channel 960 may accommodate both segments of the stretch-resistant member 970. As a further alternative, the returning segment of the stretch-resistant member 970 may be accommodated between the exterior surface of the stem 910 and the interior surface of the coil 920.

**FIG. 10A** shows a perspective view of a part of the component 900. **FIG. 10B** is a perspective view of the component 900 of **FIG. 10A****,** particularly, showing the coil 920, the stretch-resistant member 970, and the guide wire 30 coupled thereto.

As shown in **FIGS. 10A-10B****,** the component 900 may optionally further include a cover 980 covering at least a part of the stretch-resistant member 970 that is disposed around the structure 932. The cover 980 may provide some protection for the stretch-resistant member 970.

In some cases, the stem 910 and the structure 930 may be integrally formed together so that they have an unity or monolithic configuration. Also, in some cases, the stopper 950 may also be integrally formed together with the stem 910 and the structure 930 so that they have an unity or monolithic configuration. If the component 900 includes the cover 980. The cover 980 may also be integrally formed together with the stem 910 and/or the stopper 950 so that they have an unity or monolithic configuration. In other cases, the stem 910, the stopper 950, the structure 932, the cover 980, or any combination of the foregoing, may be individually formed, and may be secured to each other via an adhesive.

During a manufacturing process, the stem 910 of the component 900 may be inserted into the lumen 922 of the coil 920. Then the stretch-resistant member 970 may be inserted into the channel 960 of the stem 910. The stretch-resistant member 970 is guided around the structure 932 to form an anchor (e.g., a hook). Next, the distal portion 602 of the delivery wire 30 may be inserted into the opening 934 defined by the structure 932, and anchor against the component 900. In another variation, the stretch-resistant member 970 may be inserted into the channel 960 of the stem 910 and is guided around the structure 932 to form an anchor first - i.e., before the stem 910 of the component 900 is inserted into the lumen 922 of the coil 920. Optionally, an adhesive may be applied to secure the component 900 against the coil 920, to secure the stretch-resistant member 970 against the component 900, to secure the distal portion 602 of the delivery wire 30 against the component 900, or any combination of the foregoing. In one implementation, a blob of adhesive may be disposed around the structure 932, the stopper 950, and the proximal end 924 of the coil 920.

The stem 910, the stopper 950, and the structure 932 may be made from a same material, or different respective materials. For examples, the stem 910, the stopper 950, the structure 932, or any combination of the foregoing, may be made from polymer(s), metal(s), or alloy(s) (such as HTL, a BIO polymer, palladium, etc.). In some cases, the stopper 950, the structure 932, or any combination of the foregoing, may be made from a bio-compatible material or a 3D-printing material.

In some cases, the delivery wire 30, or at least the distal portion 602 of the delivery wire 30, may be considered to be a part of the component 900. The distal portion 602 of the delivery wire 30 is connected to a remaining portion of the delivery wire 30 via a detachable link 400. In some cases, the detachable link 400 may also be considered to be a part of the component 900. In other cases, the distal portion 602 and/or the detachable link 400 is not a part of the component 900.

In addition, in some cases, the stretch-resistant member 970 may be considered to be a part of the component 900. In such cases, the component 900 further includes the stretch-resistant member 970. In other cases, the component 900 does not include the stretch-resistant member 970.

Also, in some cases, the component 900 may optionally include the coil 920. In such cases, the coil 920 is a part of the component 900. In other cases, the coil 920 is not a part of the component 900.

Optionally, the component 900 may further optionally include a braided structure (formed by braid wires) coupled to the coil 920. The braided structure may be a part of the embolic device 100, and the braided structure may be sized for insertion into an aneurysm. Thus, in some cases, the component 900 may optionally include the embolic device 100. In other cases, the braided structure is not a part of the component 900. In further cases, the coil 920 itself may be the embolic device 100. In such cases, the component 900 does not include any braided structure.

As mentioned, the component 900 may be considered a medical device. The medica device may include only the stem 920, the stopper 950, and the structure 932. Alternatively, the medical device may optionally include the stretch-resistant member 970, the coil 920, the braid structure (formed by braid wires) coupled to the coil 920, the embolic device that comprises the braid structure and the coil 920, the distal portion 602 of the delivery wire 30, the delivery wire 30, the detachable link 400, or any combination of the foregoing.

It should be noted that the component / medical device 900 is advantageous because it provides a structural interface that connects the coil 920 with the distal portion 602 of the delivery wire 30. Also, the component / medical device 900 allows the coil 920 to be easily and efficiently connected to the delivery wire 30 via a few simple steps. In addition, because the distal portion 602 of the delivery wire 30 and the stretch-resistant member 970 are anchored to the same structure 932, the distal portion 602 of the delivery wire 30 and the stretch-resistant member 970 are anchored to each other indirectly via the structure 932. This is advantageous because the strength of the anchoring between the stretch-resistant member 970 and the delivery wire 30 is not dependent on the structural strength (e.g., tensile strength) of the structure 932. In particular, as shown in **FIG. 10A****,** the distal portion 602 of the delivery wire 30 is anchored against an interior surface of the structure that defines the opening 934, while the stretch-resistant member 970 is anchored against an exterior surface of the structure 932. Thus, the distal portion 602 of the delivery wire 30 and the stretch-resistant member are anchored against opposite surfaces of the structure 932. As a result, even if the structure 932 is damaged, the stretch-resistant member 970 and the distal portion 602 of the delivery wire 30 will still be anchored to each other.

It should be noted that the component 900 should not be limited to have the configuration shown in **FIG. 9****,** and that the component 900 may have other configurations in other cases. **FIGS. 11A-11E** illustrate variations of the component 900 of **FIG. 9****.** As shown in **FIG. 11A****,** the stem 910 may have a smooth edge or rounded finish. As shown in **FIG. 11B**, the first portion 912 of the stem 910 may include screw threads 1120 configured to allow the first portion 912 of the stem 910 to be screwed into the opening 922 of the coil 920. Also, the opening 934 defined by the structure 932 may have a non-circular shape, such as an elliptical shape. As shown in **FIG. 11C**, in some cases, the stopper 950 may not be a flange, and may be any enlarged portion that is capable of preventing the stem 910 from being inserted too deeply into the lumen 922 of the coil 920. **FIG. 11D** illustrates the component 900 of **FIG. 9****,** particularly showing the component 900 having a completed exterior finish. **FIG. 11E** illustrates the component 900 having the same configuration as that of **FIG. 11D**, except that the component 900 is made from a printing process, wherein the component 900 has rugged edges due to the printing process.

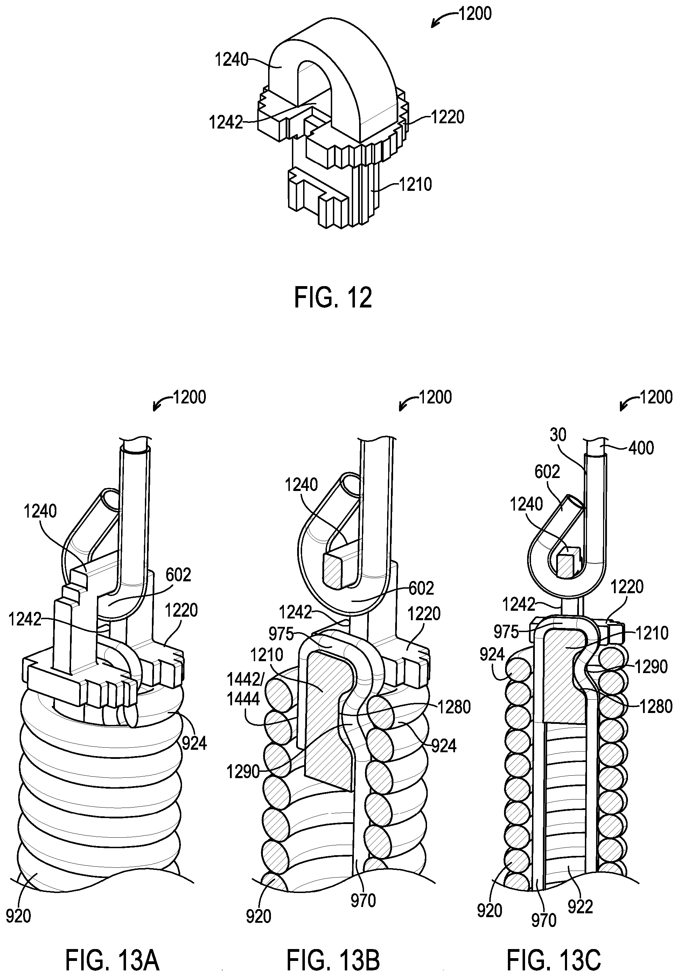

**FIG. 12** and **FIGS. 13A****-13C** illustrate another example of a component 1200 of the embolic device of **FIG. 1****.** The component 1200, which may be considered an example of a medical device, is configured to interface between a coil of the embolic device 100 (in the cases in which the embolic device 100 includes the coil) and the delivery wire 30. The component 1200 includes a stem 1210 sized for insertion into a lumen 922 of a coil 920. The component 1200 also includes a stopper 1220 coupled to, and/or extending from, the stem 1210. The stopper 1220 is proximal to a proximal end 924 of the coil 920 when the stem 1210 is inserted into the lumen 922 of the coil 920. The component 1200 also includes a loop 1240 proximal to the stopper 1220. The loop 1240 defines a loop opening 1242 sized to accommodate a distal portion 602 of a delivery wire 30, and to accommodate a part 975 of a stretch-resistant member 970.

**FIG. 12** shows the component 1200 without the coil 920, the stretch-resistant member 970, and the delivery wire 30. **FIG. 13A** is a perspective view of the component 1200 of **FIG. 12****,** particularly, showing the coil 920, the stretch-resistant member 970, and the guide wire 30 coupled thereto.

As shown in **FIG. 13B****,** the stretch-resistant member 970 forms a hook that terminates with an end 1442/1444 that is closer to the proximal end 924 of the coil 920 than to a distal end 926 of the coil 920 (like that shown in **FIG. 14A****).** The stretch-resistant member 970 may extend to the distal end 926 of the coil 920, form a loop there, and then extend back to the proximal end 924 of the coil 920. In such cases, both opposite ends 1442, 1444 of the stretch-resistant member 970 are located closer to the proximal end 924 of the coil 920 than to the distal end 926 of the coil 920 (like that shown in **FIG. 14A****).**

In other cases, the stretch-resistant member 970 form a loop 1446 around the stem 1210, and may extend back towards the distal end 926 of the coil 920 (like that shown in **FIGS. 13C** and **14B**).

As shown in **FIGS. 13B** and **13C****,** parts of the stretch-resistant member 970 are accommodated on opposite sides of the stem 1210. In particular, a first part of the stretch-resistant member 970 is accommodated between a first side of the stem 1210 and the coil 920, and a second part of the stretch-resistant member 970 is accommodated between a second side (opposite from the first side) of the stem 1210 and the coil 920. In other cases, the stem 1210 may have one or more channels (defined on an exterior surface of the stem 1210, or located inside the stem 1210) for accommodating respective parts of the stretch-resistant member 970.

Also, as shown in **FIG. 13B/13C****,** the stem 1210 may optionally include a recess 1280 to accommodate another part 1290 of the stretch-resistant member 970. In other cases, the stem 1210 may not include the recess 1280.

In some cases, the stem 1210 and the stopper 1220 may be integrally formed together so that they have an unity or monolithic configuration. Also, in some cases, the loop 1240 may also be integrally formed together with the stem 1210 and the stopper 1220 so that they have an unity or monolithic configuration. In other cases, the stem 1210, the stopper 1220, the loop 1240, or any combination of the foregoing, may be individually formed, and may be secured to each other via an adhesive.

During a manufacturing process, the stem 1210 of the component 1200 may be inserted into the lumen 922 of the coil 920. Then the stretch-resistant member 920 may then be inserted into the opening 1242 of the loop 1240, and be wrapped around the stem 1210 (to form a hook) to anchor the stretch-resistant member 920 against the stem 1210. Next, the distal portion 602 of the delivery wire 30 may be inserted into the opening 1242 of the loop 1240, and anchor against the component 1200. In another variation, the stretch-resistant member 970 may be inserted into the opening 1242 of the loop 1240 to anchor the stretch-resistant member 970 around the stem 1210, before the stem 1210 of the component 1200 is inserted into the lumen 922 of the coil 920. Optionally, an adhesive may be applied to secure the component 1200 against the coil 920, to secure the stretch-resistant member 970 against the component 1200, to secure the distal portion 602 of the delivery wire 30 against the component 1200, or any combination of the foregoing. In one implementation, a blob of adhesive may be disposed around the loop 1240, the stopper 1220, and the proximal end 924 of the coil 920.

Optionally, if the component 1200 includes a recess 1280 (shown in **FIGS. 13A-****13B),** after the stretch-resistant member 970 has anchored around the stem 1210, and after the stem 1210 has been inserted into the lumen 922 of the coil 920, the proximal end 924 of the coil 920 may be crimped from the exterior to press a part 1290 of the stretch-resistant member 970 into the recess 1280. This causes the part 1290 of the stretch-resistant member 970 to have a bend or a kink that is anchored within the recess 1280, thereby further anchoring the stretch-resistant member 970 against the stem 1210.

The stem 1210, the stopper 1220, and the loop 1240 may be made from a same material, or different respective materials. For examples, the stem 1210, the stopper 1220, the loop 1240, or any combination of the foregoing, may be made from polymer(s), metal(s), or alloy(s) (such as HTL, a BIO polymer, palladium, etc.). In some cases, the stem 1210, the stopper 1220, the loop 1240, or any combination of the foregoing, may be made from a bio-compatible material or a 3D-printing material.

In some cases, the delivery wire 30, or at least the distal portion 602 of the delivery wire 30, may be considered to be a part of the component 1200. The distal portion 602 of the delivery wire 30 is connected to a remaining portion of the delivery wire 30 via a detachable link 400. In some cases, the detachable link 400 may also be considered to be a part of the component 1200. In other cases, the distal portion 602 and/or the detachable link 400 is not a part of the component 1200.

In addition, in some cases, the stretch-resistant member 970 may be considered to be a part of the component 1200. In such cases, the component 1200 further includes the stretch-resistant member 970. In other cases, the component 1200 does not include the stretch-resistant member 970.

Also, in some cases, the component 1200 may optionally include the coil 920. In such cases, the coil 920 is a part of the component 1200. In other cases, the coil 920 is not a part of the component 1200.

Optionally, the component 1200 may further optionally include a braided structure (formed by braid wires) coupled to the coil 920. The braided structure may be a part of the embolic device 100, and the braided structure may be sized for insertion into an aneurysm. Thus, in some cases, the component 1200 may optionally include the embolic device 100. In other cases, the braided structure is not a part of the component 1200. In further cases, the coil 920 itself may be the embolic device 100. In such cases, the component 1200 does not include any braided structure.

As mentioned, the component 1200 may be considered a medical device. The medica device may include only the stem 1210, the stopper 1220, and the loop 1240. Alternatively, the medical device may optionally include the stretch-resistant member 970, the coil 920, the braid structure (formed by braid wires) coupled to the coil 920, the embolic device that comprises the braid structure and the coil 920, the distal portion 602 of the delivery wire 30, the delivery wire 30, the detachable link 400, or any combination of the foregoing.

It should be noted that the component / medical device 1200 is advantageous because it provides a structural interface that connects the coil 920 with the distal portion 602 of the delivery wire 30. Also, the component / medical device 1200 allows the coil 920 to be easily and efficiently connected to the delivery wire 30 via a few simple steps. In addition, because the distal portion 602 of the delivery wire 30 and the stretch-resistant member 970 are both inserted into the same opening 1242 of the loop 1240, the component 1200 does not require multiple different openings to respectively accommodate the delivery wire 30 and the stretch-resistant member 970.

It should be noted that the component 1200 should not be limited to have the configuration shown in **FIG. 12****,** and that the component 1200 may have other configurations in other cases.

**FIG. 14A** illustrates an example of a part of the embolic device 100 of **FIG. 1****.** As shown in the figure, the embolic device 100 includes the coil 920 having the proximal end 924 and a distal end 926. The embolic device 100 also includes a braided structure 1400 coupled to the distal end 926 of the coil via a coupler 1400. In the illustrated example, the coupler 1400 includes a first coupler portion 1422 configured for placement into the lumen of the coil 920 at the distal end 926 of the coil 920. The coupler 1400 also includes a second coupler portion 1424 that is larger than the first coupler portion 1422. The second coupler portion 1424 is a stopper that prevents the first coupler portion 1422 from being inserted too deeply into the lumen of the coil 920. The coupler 1420 has an opening 1426 configured to accommodate and to grip against the braid wires of the braided structure 1400.

In the illustrated example, the stretch-resistant member 970 has opposite ends 1442, 1444 that are closer to the proximal end 924 of the coil 920 than to the distal end 926 of the coil. In such cases, the stretch-resistant member 970 forms a loop 1446 that is closer to the distal end 926 of the coil 920 than to the proximal end 924 of the coil, and the loop is anchored distally. As shown in the figure, the loop 1446 formed by the stretch-resistant member 970 extends through the braid wires of the braided structure 1400. The proximal ends of the braid wires are coupled to an end cap 1450, which prevents the loop 1446 of the stretch-resistant member 970 from being detached from the braid wires and/or from moving proximally. Alternatively or additionally, adhesive may be applied to secure the loop 1446 of the stretch-resistant member 970 to the braid wires.

It should be noted that the coupler 1420 is not limited to having the configuration shown, and that the coupler 1420 may have other configurations in other embodiments. For example, in other cases, instead of having the cone shape, the first portion 1424 of the coupler 1420 may have other shapes. Also, in other cases, the embolic device 100 may not require the end cap 1450. For example, in other cases, the proximal ends of the braid wires of the braided structure 1400 may form a knot that anchors the loop 1446 of the stretch-resistant member 970. In further cases, the loop 1446 of the stretch-resistant member 970 does not anchor to the braid wires. Instead, the loop 1446 of the stretch-resistant member 970 may be anchored to the distal end 926 of the coil 920, and/or to the coupler 1420.

**FIG. 14B** illustrates another example of a part of the embolic device 100 of **FIG. 1****.** The embolic device 100 is the same as that of **FIG. 14A****,** except that the ends 1442, 1444 of the stretch-resistant member 970 is closer to the distal end 926 of the coil 920 than to the proximal end 924 of the coil 920. In such cases, the stretch-resistant member 970 forms the loop 1446 that is closer to the proximal end 924 of the coil 920 than to the distal end 926 of the coil 920. In such cases, the ends 1442, 1444 of the stretch-resistant member 970 may be anchored to the coupler 1420, to the distal end 926 of the coil, to the proximal ends of the braid wires of the braided structure 1400, or any combination of the foregoing.

The following items are exemplary features of embodiments described herein. Each item may be an embodiment itself or may be a part of an embodiment. One or more items described below may be combined with other item(s) in an embodiment.

Item 1: A medical device includes: a socket having one or more side wall(s) defining a cavity sized for receiving a plurality of braid wires, wherein the socket also has an end wall coupled to, and/or extending from, the one or more side wall(s); and a first anchor on an interior surface of the one or more side wall(s) of the socket, wherein the first anchor is configured to press against at least one of the braid wires after the socket has received the braid wires.

Item 2: Optionally, the medical device may further include a loop coupled to the end wall of the socket, the loop defining a loop opening sized to accommodate a distal portion of a delivery wire.

Item 3: Optionally, the medical device may further include the delivery wire, wherein the distal portion of the delivery wire is connected to a remaining portion of the delivery wire via a detachable link.

Item 4: Optionally, the loop and the cavity are on opposite respective sides of the end wall.

Item 5: Optionally, the one or more side wall(s) comprises a plurality of side walls.

Item 6: Optionally, the one or more side wall(s) comprises only a single circumferential side wall defining the cavity.

Item 7: Optionally, the medical device further includes the braid wires, wherein the braid wires are parts of a braided structure.

Item 8: Optionally, the medical device is an embolic device, and wherein the braided structure is sized for insertion into an aneurysm.

Item 9: Optionally, the braid wires comprise at least 3 braid wires.

Item 10: Optionally, the medical device further includes an adhesive connecting to the socket and the braid wires.

Item 11: Optionally, the first anchor comprises a strut or a barb.

Item 12: Optionally, the medical device further includes a second anchor on the interior surface of the one or more side wall(s) of the socket.

Item 13: Optionally, the first anchor and the second anchor are in a row oriented in a direction that is parallel to a longitudinal axis of the socket.

Item 14: Optionally, the socket is made from HTL, a BIO polymer, palladium, a bio-compatible material, or a 3D-printing material.

Item 15: Optionally, the medical device further includes one or more additional anchors, wherein the first anchor and the one or more additional anchors are configured to allow the braid wires to be inserted into the socket with an insertion force, and to resist a removal force to prevent the braid wires from being removed from the socket, wherein the removal force is higher than the insertion force.

Item 16: A medical device includes: a stem sized for insertion into a lumen of a coil; a stopper coupled to, and/or extending from, the stem, wherein the stopper is proximal to a proximal end of the coil when the stem is inserted into the lumen of the coil; and a structure outside the lumen of the coil, the structure defining an opening sized to accommodate a distal portion of a delivery wire, and wherein the stem comprises a channel configured to accommodate a first part of a stretch-resistant member when a second part of the stretch-resistant member is disposed around the structure that defines the opening.

Item 17: Optionally, the medical device further includes the stretch-resistant member.

Item 18: Optionally, the medical device further includes the delivery wire, wherein the distal portion of the delivery wire is connected to a remaining portion of the delivery wire via a detachable link.

Item 19: Optionally, the medical device further includes a braided structure coupled to the coil, wherein the braided structure is sized for insertion into an aneurysm.

Item 20: Optionally, the medical device further includes the coil.

Item 21: A medical device includes: a stem sized for insertion into a lumen of a coil; a stopper coupled to, and/or extending from, the stem, wherein the stopper is proximal to a proximal end of the coil when the stem is inserted into the lumen of the coil; and a loop proximal to the stopper, the loop defining a loop opening sized to accommodate a distal portion of a delivery wire, and to accommodate a part of a stretch-resistant member.

Item 22: Optionally, the stem comprises a recess to accommodate another part of the stretch-resistant member.

Item 23: Optionally, the medical device further includes the stretch-resistant member.

Item 24: Optionally, the medical device further includes the delivery wire, wherein the distal portion of the delivery wire is connected to a remaining portion of the delivery wire via a detachable link.

Item 25: Optionally, the medical device further includes a braided structure coupled to the coil, wherein the braided structure is sized for insertion into an aneurysm.

Item 26: Optionally, the medical device further includes the coil.

Although particular embodiments have been shown and described, it will be understood that it is not intended to limit the claimed inventions to the preferred embodiments, and it will be obvious to those skilled in the art that various changes and modifications may be made without department from the spirit and scope of the claimed inventions. The specification and drawings are, accordingly, to be regarded in an illustrative rather than restrictive sense. The claimed inventions are intended to cover alternatives, modifications, and equivalents.

## Claims

1. A medical device comprising:
a stem (910) sized for insertion into a lumen (922) of a coil (920);
a stopper (950) coupled to, and/or extending from, the stem, wherein the stopper (950) is proximal to a proximal end (924) of the coil (920) when the stem (910) is inserted into the lumen (922) of the coil (920); and
a structure (932) outside the lumen (922) of the coil (920), the structure (932) defining an opening (934) sized to accommodate a distal portion (602) of a delivery wire (30), and wherein the stem (910) comprises a channel (960) configured to accommodate a first part (972) of a stretch-resistant member (970) when a second part (974) of the stretch-resistant member (970) is disposed around the structure (932) that defines the opening (934).

2. The medical device of claim 1, further comprising the stretch-resistant member (970).

3. The medical device of claim 1, further comprising the delivery wire (30), wherein the distal portion (602) of the delivery wire (30) is connected to a remaining portion of the delivery wire via a detachable link (400).

4. The medical device of claim 1, further comprising a braided structure coupled to the coil (920), wherein the braided structure is sized for insertion into an aneurysm.

5. The medical device of claim 1, further comprising the coil (920).

6. The medical device of claim 1, wherein the stem (910) comprises screw threads (1120).

7. The medical device of claim 1, wherein the opening (934) defined by the structure (932) has a non-circular shape.

8. The medical device of claim 1, wherein the stopper (950) comprises an enlarged portion configured to prevent the stem (910) from being inserted too deeply into the lumen.

9. The medical device of claim 1, wherein the stopper (950) comprises an enlarged portion that is not a flange.

10. The medical device of claim 1, wherein the medical device is made from a 3D-printing material.

11. The medical device of claim 1, further comprising a cover (980) covering the second part of the stretch-resistant member (970) that is disposed around the structure (932).

12. The medical device of claim 11, wherein the cover (980) is integrally formed together with the stem (910) and/or the stopper (950).

13. The medical device of claim 1, wherein the stretch-resistant member (970) comprises a third part extending form the second part, wherein the channel (960) is configured to accommodate the third part of the stretch-resistant member (970).

14. The medical device of claim 1, wherein the stretch-resistant member (970) comprises a third part extending form the second part, wherein the stem comprises another channel configured to accommodate the third part of the stretch-resistant member (970).

15. The medical device of claim 1, wherein the stretch-resistant member (970) comprises a third part extending from the second part, wherein the third part of the stretch-resistant member (970) is accommodated between an exterior surface of the stem (910) and an interior surface of the coil (920).
